# EUROPEAN PATENT APPLICATION

(11) **EP 4 851 183 A1**
(43) Date of publication of application: **09.12.2026**
(21) Application number: 25747986.5
(22) Date of filing: 27.01.2025
(51) Int. Cl.: C07K 14/605, A61K 38/26, A61P 3/10

(54) **GLUCAGON-LIKE PEPTIDE 2 DERIVATIVE AND USE THEREOF**

(30) Priority: 31.01.2024 CN 202410136239
(71) Applicant: Hangzhou Sciwind Biosciences Co., Ltd., Hangzhou, Zhejiang 310018 (CN); Sciwind Biosciences (Beijing) Co., Ltd., Beijing 100176 (CN)
(72) Inventor: WU, Xinle, Beijing 100176 (CN); ZOU, Haixia, Beijing 100176 (CN); ZHU, Rui, Beijing 100176 (CN)
(74) Representative: Engelhard, Markus
(86) International application number: PCT/CN2025/075381
(87) International publication number: WO 2025/162422

(57) **Abstract**

Provided are a glucagon-like peptide 2 derivative and the use thereof. The glucagon-like peptide 2 derivative has an amino acid sequence of HGDGSFSDEMNTILDX₁₆LAARDFIX₂₄WLIQTKITD, wherein X₁₆ is selected from K or L, X₂₄ is selected from N or K, at least one of X₁₆ and X₂₄ is K, and amino acid K residues at positions 16 and/or 24 of the derivative are linked to a fatty acid side chain. The glucagon-like peptide 2 derivative can be used for promoting small intestine growth, and preventing and/or treating obesity and gastric and intestinal-related disorders.

## Description

### TECHNICAL FIELD

The present disclosure pertains to the field of biomedicine and relates to a glucagon-like peptide-2 (GLP-2) derivative and use thereof.

### BACKGROUND

Glucagon-like peptide-2 (GLP-2) is a polypeptide secreted by intestinal L cells upon food stimulation. GLP-2 exerts the effect of increasing villi and crypt growth by binding to GLP-2 receptors located in enteroendocrine cells and enteroneuronal cells. GLP-2 can also increase intestinal and portal venous blood flow, inhibit gastric emptying and gastric acid secretion, and the like, and ultimately enhance intestinal absorption of nutrients.

Short bowel syndrome (SBS) is a rare and debilitating disease caused by loss of functional small intestine and afflicts three million people worldwide. In adults, short bowel syndrome is generally caused by intestinal resection due to trauma, malignancies, chronic enteritis, or the like. Since the residual intestine is very short, the ability to absorb nutrients, fluids, and the like is lost. Short bowel syndrome may lead to intestinal failure (IF), i.e., reduction of intestinal function to the minimum level required for absorption of nutrients, water, and electrolytes in the human body, thereby causing complications such as diarrhea, dehydration, and malnutrition. The quality of life and life expectancy of patients with short bowel syndrome are thus severely affected. In order to obtain sufficient nutrition and energy support, intravenous infusion of nutritional solutions is a conventional treatment means. However, this method is highly inconvenient and unfriendly. In 2012, teduglutide (trade name: Gattex) developed by Takeda was the only GLP-2 analog approved for marketing in the European Union for the treatment of adult SBS. In 2019, the indication was expanded to include pediatric SBS patients aged 1 year or older. Teduglutide can help the intestine to absorb more nutrients and reduce the frequency and volume of parenteral nutrition, and can be considered as the first disease-modifying therapy. However, this drug has a half-life of only about 1.3 h (https://www.accessdata.fda.gov/drugsatfda_docs/label/2012/203441Orig1s000lbl.pdf), and requires subcutaneous injection daily. To further reduce the frequency of administration, some long-acting GLP-2 receptor agonists are currently in the development stage, including apraglutide (VectiveBio) and glepaglutide (Zealand Pharma) with twice-weekly or once-weekly injection in phase III clinical trials, as well as HM15912 (Hanmi) with once-monthly injection in phase II clinical trials.

GLP-2 also has an anti-inflammatory function, which enables it to become a potential target for the treatment of inflammatory bowel disease (IBD) (including Crohn's disease and ulcerative colitis). In addition, GLP-2 regulates intestinal motility and is potentially used for the regulation of irritable bowel syndrome (IBS). GLP-2 is also used in studies on graft-versus-host disease (GvHD), chemotherapy-induced diarrhea, and the like.

### SUMMARY

The present disclosure provides a long-acting GLP-2 derivative. Compared with the prior art, the long-acting GLP-2 derivative has higher GLP-2R activation activity and a longer half-life, which can greatly reduce the administration frequency and improve patient compliance and experience. In addition, by combining with NAC salt (N-[8-(2-hydroxybenzoyl)amino]octanoate) delivery technology, oral administration of the GLP-2 derivative is also achieved, thereby further improving patient compliance and convenience.

In a first aspect, the present disclosure provides a glucagon-like peptide-2 derivative or a pharmaceutically acceptable salt thereof, wherein the amino acid sequence of the glucagon-like peptide-2 derivative is:

HGDGSFSDEMNTILDX₁₆LAARDFIX₂₄WLIQTKITD,

wherein X₁₆ is selected from K and L, X₂₄ is selected from N and K, and at least one of X₁₆ and X₂₄ is K;
a fatty acid side chain is linked to the amino acid K residue at position 16 and/or 24 of the derivative,
wherein the linkage is a direct linkage or an indirect linkage (e.g., a linkage via a linker).

In some embodiments, in the glucagon-like peptide-2 derivative or the pharmaceutically acceptable salt thereof described above, X₁₆ is K, X₂₄ is N, and the amino acid sequence of the glucagon-like peptide-2 derivative is set forth in SEQ ID NO: 3.

In some embodiments, in the glucagon-like peptide-2 derivative or the pharmaceutically acceptable salt thereof described above, X₁₆ is L, X₂₄ is K, and the amino acid sequence of the glucagon-like peptide-2 derivative is set forth in SEQ ID NO: 4.

In some embodiments, in the glucagon-like peptide-2 derivative or the pharmaceutically acceptable salt thereof according to any one of the embodiments described above, the derivative is linked to the fatty acid side chain via the ε amino group of the amino acid K residue at position 16 and/or 24.

In some embodiments, in the glucagon-like peptide-2 derivative or the pharmaceutically acceptable salt thereof according to any one of the embodiments described above, the fatty acid side chain is selected from one or more of 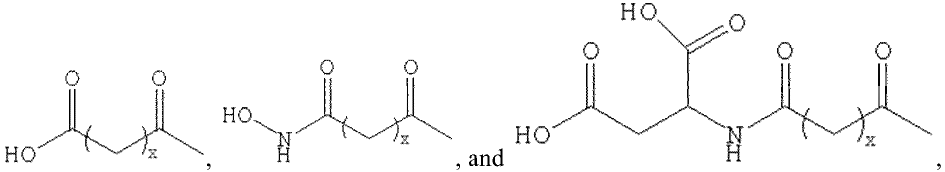
wherein x is any integer from 4 to 38;
preferably, the fatty acid side chain is selected from one or more of:

   HOOC(CH₂)₁₄CO-, HOOC(CH₂)₁₅CO-, HOOC(CH₂)₁₆CO-, HOOC(CH₂)₁₇CO-, HOOC(CH₂)₁₈CO-, HOOC(CH₂)₁₉CO-, HOOC(CH₂)₂₀CO-, HOOC(CH₂)₂₁CO-, and HOOC(CH₂)₂₂CO-;
more preferably, the fatty acid side chain is HOOC(CH₂)₁₆CO-.

In some embodiments, in the glucagon-like peptide-2 derivative or the pharmaceutically acceptable salt thereof according to any one of the embodiments described above, the fatty acid side chain is linked to the amino acid K residue at position 16 and/or 24 of the derivative via a linker.

In some embodiments, in the glucagon-like peptide-2 derivative or the pharmaceutically acceptable salt thereof described above, the linker is selected from one or more of 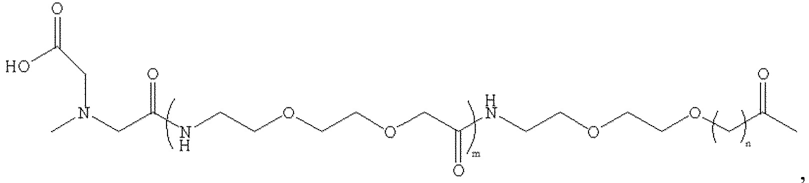 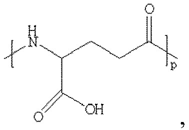 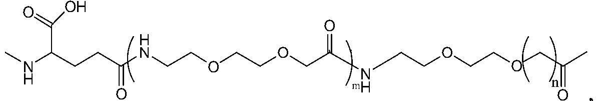 or 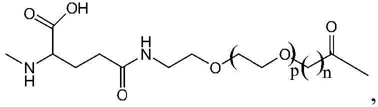 wherein m is 0, 1, 2, or 3; n is 1 or 2; p is any integer from 1 to 5;
preferably, the linker is: 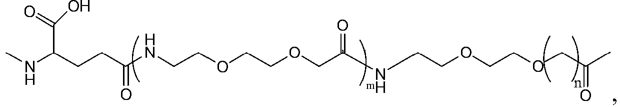 wherein m is 0, 1, 2, or 3, and n is 1; more preferably, m is 1, and n is 1.

In some embodiments, the glucagon-like peptide-2 derivative is the polypeptide derivative M2 of the present disclosure, the amino acid sequence of which is set forth in SEQ ID NO: 3, and the ε amino group on the amino acid K residue at position 16 is linked to (2-(2-(2-aminoethoxy)ethoxy)acetyl)₂-(γGlu)-CO-(CH₂)₁₆-CO₂H (18-{[(23S)-23-carboxy-2,11,20-trioxo-10,19-diaza-4,7,13,16-tetraoxatricos-23-yl]amino}-18-oxooctadecanoic acid) via an amide bond; (2-(2-(2-aminoethoxy)ethoxy)acetyl)₂-(yGlu)-CO-(CH₂)₁₆-CO₂H has a structural formula represented by formula (I):

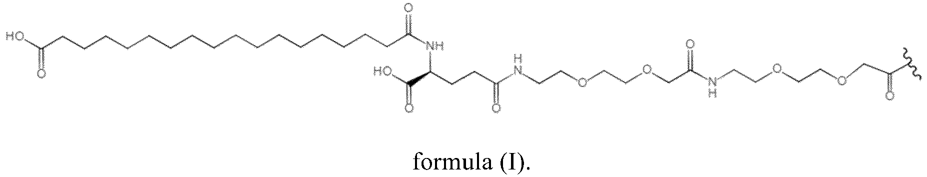

In some embodiments, the glucagon-like peptide-2 derivative is the polypeptide derivative M3 of the present disclosure, the amino acid sequence of which is set forth in SEQ ID NO: 4, and the ε amino group on the amino acid K residue at position 24 is linked to (2-(2-(2-aminoethoxy)ethoxy)acetyl)₂-(γGlu)-CO-(CH₂)₁₆-CO₂H via an amide bond; (2-(2-(2-aminoethoxy)ethoxy)acetyl)₂-(γGlu)-CO-(CH₂)₁₆-CO₂H also has a structural formula represented by formula (I).

In a second aspect, the present disclosure provides a method for preparing the glucagon-like peptide-2 derivative or the pharmaceutically acceptable salt thereof according to any one of the embodiments described above, wherein the preparation method comprises a step of preparing the glucagon-like peptide-2 derivative using a chemical method and/or a biological method;
preferably, the chemical method comprises liquid-phase or solid-phase polypeptide synthesis; the biological method comprises a molecular biological method and/or a cell biological method.

In a third aspect, the present disclosure provides a pharmaceutical composition comprising the glucagon-like peptide-2 derivative or the pharmaceutically acceptable salt thereof according to any one of the embodiments described above, and a pharmaceutically acceptable excipient;
the pharmaceutical composition of the present disclosure can be administered by means of any suitable route known in the art, the route including, but not limited to: oral, nasal, intradermal, subcutaneous, intravenous, intramuscular, intrabronchial, intrapleural, intraperitoneal, intraarterial, lymphatic, and/or cerebrospinal routes;
preferably, the pharmaceutical composition is in the form of a solid, liquid, or semisolid;
preferably, the pharmaceutically acceptable excipient comprises one or more pharmaceutically acceptable lubricants, such as magnesium stearate;
preferably, the pharmaceutical composition is an oral delivery composition; preferably, the oral delivery composition is in the form of a solid, liquid, or semisolid, more preferably a solid, and further preferably an oral tablet;
preferably, the oral delivery composition further comprises an oral absorption enhancer, and the oral absorption enhancer is an ingredient capable of improving the oral absorption of an active pharmaceutical ingredient and is selected from one or more of the following: an NAC salt, decanoate, Cu, Zn, or Fe ions, a reducing agent, tetrasodium ethylenediaminetetraacetate, sodium phosphate, tris(hydroxymethyl)aminomethane, and lysine; the reducing agent is preferably ascorbic acid; preferably, the oral absorption enhancer is an NAC salt (N-[8-(2-hydroxybenzoyl)amino]octanoate), and the NAC salt may be one or more of a sodium salt (SNAC) and a potassium salt (PNAC).

In some embodiments, the pharmaceutical composition is an oral tablet comprising the glucagon-like peptide-2 derivative or the pharmaceutically acceptable salt thereof according to any one of the embodiments described above, an NAC salt, and magnesium stearate; in some embodiments, the oral tablet comprises the glucagon-like peptide-2 derivative or the pharmaceutically acceptable salt thereof according to any one of the embodiments described above, PNAC, and magnesium stearate.

In some embodiments, the pharmaceutical composition of the present disclosure further comprises one or more additional active pharmaceutical ingredients. In some embodiments, the active pharmaceutical ingredient may exhibit beneficial effects on the prevention and/or treatment of obesity and/or a gastrointestinal disorder, and may be, for example, an anti-inflammatory active ingredient;
preferably, the gastrointestinal disorder is ulcer, digestive disorder, malnutrition, malabsorption syndrome, short bowel syndrome, blind loop syndrome, inflammatory bowel disease, celiac sprue, tropical sprue, hypogammaglobulinemia-type sprue, small intestine injury, chemotherapy-induced diarrhea/mucositis, irritable bowel syndrome, or graft-versus-host disease; preferably, the inflammatory bowel disease is Crohn's disease or ulcerative colitis.

In some embodiments, the pharmaceutical composition of the present disclosure may be used in combination with one or more additional pharmaceutical compositions. In some embodiments, different pharmaceutical compositions may be administered to a patient in need thereof simultaneously, sequentially, or separately. In some embodiments, different pharmaceutical compositions are administered to a patient in need thereof according to a time sequence; for example, the pharmaceutical compositions are each administered once, twice, three times, or more times per day simultaneously, sequentially, or separately within 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 1 month, 2 months, 3 months, or longer.

In a fourth aspect, the present disclosure provides use of the glucagon-like peptide-2 derivative or the pharmaceutically acceptable salt thereof according to any one of the embodiments described above, or the pharmaceutical composition according to any one of the embodiments described above in the manufacture of a medicament for promoting small intestine growth, preventing and/or treating obesity, and preventing and/or treating a gastrointestinal disorder;
preferably, the gastrointestinal disorder is ulcer, digestive disorder, malnutrition, malabsorption syndrome, short bowel syndrome, blind loop syndrome, inflammatory bowel disease, celiac sprue, tropical sprue, hypogammaglobulinemia-type sprue, small intestine injury, chemotherapy-induced diarrhea/mucositis, irritable bowel syndrome, or graft-versus-host disease; preferably, the inflammatory bowel disease is Crohn's disease or ulcerative colitis;
preferably, the medicament is an oral drug;
preferably, the oral drug is in the form of a solid, liquid, or semisolid, more preferably a solid, and further preferably an oral tablet;
preferably, promoting small intestine growth comprises promoting increases in small intestine weight and small intestine thickness.

In a fifth aspect, the present disclosure provides the glucagon-like peptide-2 derivative or the pharmaceutically acceptable salt thereof according to any one of the embodiments described above or the pharmaceutical composition according to any one of the embodiments described above for use in therapy.

In a sixth aspect, the present disclosure further provides a method for promoting small intestine growth, preventing and/or treating obesity, and preventing and/or treating a gastrointestinal disorder, comprising administering to a patient in need thereof a therapeutically effective amount of the glucagon-like peptide-2 derivative or the pharmaceutically acceptable salt thereof according to any one of the embodiments described above or the pharmaceutical composition according to any one of the embodiments described above;
preferably, the gastrointestinal disorder is ulcer, digestive disorder, malnutrition, malabsorption syndrome, short bowel syndrome, blind loop syndrome, inflammatory bowel disease, celiac sprue, tropical sprue, hypogammaglobulinemia-type sprue, small intestine injury, chemotherapy-induced diarrhea/mucositis, irritable bowel syndrome, or graft-versus-host disease; preferably, the inflammatory bowel disease is Crohn's disease or ulcerative colitis;
preferably, promoting small intestine growth comprises promoting increases in small intestine weight and small intestine thickness.

The medicament of the present disclosure can be administered to the patient (e.g., a mammal, such as a human) by means of any suitable route known in the art, the route including but not limited to: oral, nasal, intradermal, subcutaneous, intravenous, intramuscular, intrabronchial, intrapleural, intraperitoneal, intraarterial, lymphatic, and/or cerebrospinal routes.

In some embodiments, in the method according to any one of the embodiments described above, the administration frequency of the glucagon-like peptide-2 derivative or the pharmaceutically acceptable salt thereof or the pharmaceutical composition is once or more times every day, every week, every two weeks, every three weeks, every month, every 2 months, every 3 months, every 4 months, every 5 months, every 6 months, every 7 months, every 8 months, every 9 months, every 10 months, every 11 months, or every 12 months, e.g., 1 time, 2 times, 3 times, 4 times, 5 times, 6 times, 7 times, 8 times, 9 times, 10 times, 11 times, 12 times, 13 times, 14 times, 15 times, 16 times, 17 times, 18 times, 19 times, 20 times, 21 times, 22 times, 23 times, 24 times, 25 times, 26 times, 27 times, 28 times, 29 times, 30 times, 31 times, 32 times, 33 times, 34 times, 35 times, 36 times, 37 times, 38 times, 39 times, 40 times, 41 times, 42 times, 43 times, 44 times, 45 times, 46 times, 47 times, 48 times, 49 times, or 50 times every day, every week, every two weeks, every three weeks, every month, every 2 months, every 3 months, every 4 months, every 5 months, every 6 months, every 7 months, every 8 months, every 9 months, every 10 months, every 11 months, or every 12 months.

In some embodiments, in the method according to any one of the embodiments described above, the total number of administrations of the glucagon-like peptide-2 derivative or the pharmaceutically acceptable salt thereof or the pharmaceutical composition may be 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50.

The present disclosure further provides a kit comprising the glucagon-like peptide-2 derivative or the pharmaceutically acceptable salt thereof or the pharmaceutical composition according to any one of the embodiments described above, and optionally instructions for use.

The present disclosure has the following beneficial effects:
1. Compared with the prior art, the polypeptide derivative of the present disclosure greatly improves the GLP-2R activation activity and provides an efficient pharmaceutical ingredient for preventing and/or treating GLP-2R-related diseases.
2. The half-life of the polypeptide derivative of the present disclosure is significantly prolonged, thereby reducing the administration frequency and improving patient compliance and experience.
3. Meanwhile, the polypeptide derivative of the present disclosure can be effectively adapted for use with oral delivery agents, and thus can be applied to oral drugs. For example, by combining with NAC salt (N-[8-(2-hydroxybenzoyl)amino]octanoate) delivery technology, oral absorption of the polypeptide derivative is achieved, thereby further improving patient compliance and convenience.

The glucagon-like peptide-2 derivative of the present disclosure can be used for promoting small intestine growth, and preventing and/or treating obesity and a gastrointestinal disorder.

### BRIEF DESCRIPTION OF THE DRAWINGS

FIG. 1 shows the rat pharmacokinetic profile of Example 3.
FIG. 2 shows the beagle dog pharmacokinetic profile of Example 4.
FIG. 3 shows the experimental results of the small intestine growth-promoting pharmacodynamic study of Example 5.
FIG. 4 shows the beagle dog pharmacokinetic profile after oral administration of Example 6.

### DETAILED DESCRIPTION

The experimental methods used in the following examples are all conventional methods, unless otherwise stated.

The materials, reagents, and the like used in the following examples can be commercially available, unless otherwise stated.

The present disclosure will be further illustrated with reference to the following specific examples.

It should be understood that the following examples are merely intended to illustrate the present disclosure rather than limit the scope of the present disclosure.

### Definitions

Unless otherwise stated, the terms used herein have the following definitions.

Herein, conventional single-letter and three-letter codes for natural amino acids, and the generally accepted three-letter codes for other amino acids, e.g., norleucine (Nle) are used. All amino acid residues in the peptides of the present disclosure are in an L-configuration, unless otherwise stated. The terms "polypeptide", "peptide", and "protein" are used interchangeably. "Polypeptide", "peptide", or "protein" refers to any chain comprising two or more amino acids, regardless of post-translational modifications (e.g., glycosylation or phosphorylation), including naturally occurring or non-naturally occurring (e.g., artificially synthesized) amino acids or amino acid analogs, wherein the amino acids in any chain are covalently linked via peptide bonds.

The term "fatty acid-modified polypeptide" or "polypeptide derivative" refers to a polypeptide with a fatty acid side chain modification.

The term "include", "comprise", or "contain" should be interpreted as including the specified components but not excluding any other components.

The terms "patient", "subject", and "individual" are used interchangeably and include human and non-human animals, among which the non-human animals include mammals, e.g., monkeys, rats, mice, cows, pigs, goats, sheep, dogs, and cats.

When "administering" and "treating" involve an animal, a human, an experimental subject, a cell, a tissue, an organ, or a biological fluid, the terms refer to contacting an exogenous drug, a therapeutic agent, a diagnostic agent, or a composition with the animal, human, subject, cell, tissue, organ, or biological fluid. "Administering" and "treating" may refer to, for example, a treatment method, a pharmacokinetic method, a diagnostic method, a research method, and an experimental method. Treating a cell encompasses contacting a reagent with the cell, as well as contacting a reagent with a fluid, wherein the fluid is in contact with the cell. "Administering" and "treating" also mean *in vitro* and *ex vivo* treatment of a cell, for example, by a reagent, a diagnostic agent, a binding composition, or other cells.

"Preventing" or "treating" used herein includes a postponement of development of the symptoms associated with a disease and/or a reduction in the severity of these symptoms that will or are expected to develop with the disease. The term also includes ameliorating existing symptoms, preventing additional symptoms, and ameliorating or preventing the underlying causes of these symptoms. Thus, the term indicates that a beneficial result has been imparted to a vertebrate subject with a disease, such as a human.

The term "therapeutically effective amount" or "effective amount" used herein refers to an amount of the GLP-2 derivative or the pharmaceutically acceptable salt thereof that, when administered alone or in combination with an additional therapeutic agent to a cell, tissue, or subject, is effective in preventing or ameliorating the disease or disorder to be treated. The therapeutically effective amount further refers to an amount of the GLP-2 derivative or the pharmaceutically acceptable salt thereof that is sufficient to cause amelioration of symptoms, e.g., treatment, healing, prevention, or amelioration of a related medical state, or an increase in the rate of treatment, healing, prevention, or amelioration of the disorder. The effective amount for a specific subject may vary based on a variety of factors, such as the disease to be treated, the overall health status of the patient, the method, route, and dose of administration, and the severity of side effects. The effective amount may be the maximum dose or administration regimen that avoids significant side effects or toxic effects. When an active ingredient is administered to an individual alone, the therapeutically effective amount refers to that individual ingredient. When a combination is administered, the therapeutically effective amount refers to the combined amount of the active ingredients that produces a therapeutic effect, regardless of whether they are administered in combination, sequentially, or simultaneously. The therapeutically effective amount will alleviate symptoms generally by at least 10%, generally by at least 20%, preferably by at least about 30%, more preferably by at least 40%, and most preferably by at least 50%.

### GLP-2 derivative and pharmaceutically acceptable salt thereof

The present disclosure provides a glucagon-like peptide-2 derivative (GLP-2 derivative) with significantly improved GLP-2R activation activity, significantly prolonged half-life, and significantly improved plasma exposure level as compared with apraglutide, which is a fatty acid-modified polypeptide or a polypeptide derivative.

As demonstrated by experiments, the polypeptide derivatives M2 and M3 of the present disclosure have higher GLP-2R activation activity, longer half-life, and higher plasma exposure level than apraglutide.

The amino acid sequence of the GLP-2 derivative M2 of the present disclosure is set forth in SEQ ID NO: 3, and the ε amino group on the amino acid K residue at position 16 is linked to (2-(2-(2-aminoethoxy)ethoxy)acetyl)₂-(γGlu)-CO-(CH₂)₁₆-CO₂H (18-{[(23S)-23-carboxy-2,11,20-trioxo-10,19-diaza-4,7,13,16-tetraoxatricos-23-yl]amino}-18-oxooctadecanoic acid) via an amide bond; (2-(2-(2-aminoethoxy)ethoxy)acetyl)₂-(γGlu)-CO-(CH₂)₁₆-CO₂H has a structural formula represented by formula (I) herein.

The amino acid sequence of the GLP-2 derivative M3 of the present disclosure is set forth in SEQ ID NO: 4, and the ε amino group on the amino acid K residue at position 24 is linked to (2-(2-(2-aminoethoxy)ethoxy)acetyl)₂-(γGlu)-CO-(CH₂)₁₆-CO₂H (18-{[(23S)-23-carboxy-2,11,20-trioxo-10,19-diaza-4,7,13,16-tetraoxatricos-23-yl]amino}-18-oxooctadecanoic acid) via an amide bond; (2-(2-(2-aminoethoxy)ethoxy)acetyl)₂-(γGlu)-CO-(CH₂)₁₆-CO₂H has a structural formula represented by formula (I) herein.

It should be understood that the GLP-2 derivative of the present disclosure may also be provided in the form of a salt. Pharmaceutically acceptable salts include salts in the form of anions and salts in the form of cations. Some examples of salts in the form of anions include hydrochloride, citrate, chloride salts, and acetate. Preferably, the salt is acetate. Some examples of salts in the form of cations include salts in which the cations are selected from the following: alkali metals (e.g., sodium and potassium), alkaline earth metals (e.g., calcium), and the like.

In addition, the GLP-2 derivative of the present disclosure may further form a coordination complex with metal ions (e.g., Mn²⁺ and Zn²⁺), thereby existing in the form of a complex. Due to the presence of hydroxy or carboxylic acids in the GLP-2 derivative of the present disclosure, the derivative may further react with suitable carboxylic acids or alcohols to form esters, thereby existing in the form of an ester. The GLP-2 derivative of the present disclosure may further exist in the form of a prodrug, which may be converted into one of the parent compounds *in vivo* or *in vitro*. Generally, at least one biological activity of the GLP-2 derivative may be reduced in the prodrug form and can be activated by conversion of the prodrug to release the GLP-2 derivative or a metabolite thereof. Some examples of prodrugs include the use of protecting groups that can be removed *in situ* to release active compounds or used to inhibit drug clearance *in vivo*.

### Synthesis of GLP-2 derivative

The GLP-2 derivative of the present disclosure can be prepared using a chemical method and/or a biological method. The preparation is preferably carried out using the chemical method, e.g., using a liquid-phase or solid-phase polypeptide synthesis means to synthesize the GLP-2 derivative of the present disclosure. The biological method includes a molecular biological method and a cell biological method.

The method for preparing the GLP-2 derivative of the present disclosure may comprise the following steps:
synthesizing the GLP-2 derivative of the present disclosure step by step or through fragment assembly according to a polypeptide sequence by the liquid-phase or solid-phase polypeptide synthesis means; or
introducing a nucleic acid construct encoding the polypeptide sequence of the GLP-2 derivative into a host cell, and then after culturing for a period of time in a certain condition, obtaining a polypeptide product of the GLP-2 derivative from the host cell culture, for example, by expressing the polypeptide in a prokaryotic host (e.g., *Escherichia coli*) or a eukaryotic host (e.g., yeast, higher plant or animal) using recombinant technology, followed by modification of the polypeptide with a fatty acid side chain to obtain the GLP-2 derivative of the present disclosure; or
expressing the polypeptide product of the GLP-2 derivative in a cell-free system using a nucleic acid construct encoding the polypeptide sequence of the glucagon-like peptide-2 derivative, and modifying the polypeptide with a fatty acid side chain to obtain the GLP-2 derivative of the present disclosure.

In some embodiments, the GLP-2 derivative of the present disclosure is prepared by solid-phase polypeptide synthesis on a suitable resin. The steps of solid-phase polypeptide synthesis are well known in the art; for example, the solid-phase synthesis is initiated by attaching an N-terminus-protected amino acid and the carboxyl terminus to an inert solid support carrying a cleavable linker. The solid support may be any polymer that allows coupling of the original amino acid, e.g., Fmoc-Asp(OtBu)-Wang Resin. In some embodiments, Fmoc-Asp(OtBu)-Wang Resin is used as a starting material, and amino acids with Fmoc-protecting groups are sequentially coupled according to a solid-phase synthesis method to obtain a protected linear polypeptide resin, during which the Fmoc-protecting groups are sequentially removed, and peptide coupling reactions are carried out using TBTU as a condensing agent. After the protected linear polypeptide resin is obtained, the peptide is cleaved from the resin, followed by simultaneous deprotection of side-chain protecting groups and coupling of building blocks to obtain a fatty acid-modified polypeptide resin. The peptide is then cleaved from the resin, separated and purified by a chromatographic column, and subsequently lyophilized to obtain a refined polypeptide derivative in the form of a powder.

In some embodiments, the polypeptide sequence of the GLP-2 derivative of the present disclosure is prepared using recombinant technology. In this case, the present disclosure further provides a nucleic acid molecule encoding the polypeptide sequence of the GLP-2 derivative of the present disclosure, and the nucleotide sequence of the nucleic acid molecule may be a codon-optimized sequence based on the host into which it is to be introduced. The nucleic acid molecule may be a DNA fragment or an RNA fragment, and may generally be obtained by amplification using a PCR instrument or by artificial synthesis.

In some embodiments, the polypeptide sequence of the GLP-2 derivative of the present disclosure is prepared using recombinant technology. In this case, the present disclosure further provides a recombinant vector comprising the nucleic acid molecule described above. The recombinant vector comprises a cloning vector and an expression vector, wherein the cloning vector is used for replicating related sequences, and the expression vector is used for expressing related genes. The vector may be any vector commonly used in the art, such as a plasmid, a phage, a cosmid, a minichromosome, or a virus. In addition to the nucleic acid encoding the polypeptide sequence of the GLP-2 derivative described above, the expression vector may comprise not only a promoter for initiating transcription of the gene encoding the polypeptide sequence, but also a signal peptide sequence, a terminator for terminating transcription of the gene encoding the polypeptide sequence, and an enhancer sequence.

The method for constructing the recombinant expression vector may be any known method. The promoter described above, the nucleic acid of the gene encoding the polypeptide sequence, and other DNA segments (e.g., terminators or enhancers) if present may be introduced in a predetermined order into an appropriately selected vector serving as the basis. For example, the recombinant vector may be constructed by using a restriction endonuclease, a ligase, and the like. In some embodiments, the polypeptide sequence of the GLP-2 derivative of the present disclosure is prepared using recombinant technology. In this case, the present disclosure further provides a recombinant cell comprising the recombinant vector described above. The recombinant cell is induced or not induced to express the polypeptide sequence of the GLP-2 derivative of the present disclosure. In some embodiments, the method for constructing the recombinant cell comprises the following steps: transforming the recombinant expression vector into an expression host cell, culturing the expression host cell, and adding an inducer to induce (if necessary) expression to obtain the polypeptide sequence of the GLP-2 derivative described above. Further, the expression host cell is a prokaryotic cell or a eukaryotic cell, such as *Escherichia coli*, yeast, a plant cell, or an animal cell.

More specifically, the method for constructing the recombinant cell described above comprises the following steps:
(1) amplification of a gene encoding the polypeptide sequence of the GLP-2 derivative;
(2) construction of a recombinant expression vector;
(3) transformation or transfection of the recombinant expression vector into an expression host cell; and
(4) optionally, screening to obtain positive clones.

The polypeptide of the present disclosure may be secreted extracellularly, or expressed on the cell surface or intracellularly.

In some embodiments, the preparation of the polypeptide sequence of the GLP-2 derivative of the present disclosure using recombinant technology comprises the following steps:
(1) culturing the recombinant cell described above and adding an inducer to induce (if necessary) expression of the polypeptide sequence of the GLP-2 derivative of the present disclosure to obtain a cell culture; and
(2) optionally, separating and purifying the polypeptide sequence of the GLP-2 derivative of the present disclosure from the cell culture (e.g., cells or cell culture supernatant).

### Biological activity

The GLP-2 derivative of the present disclosure has activation activity for the receptor GLP-2R and is a GLP-2R agonist.

In the present disclosure, the EC₅₀ value is used as a numerical measure of agonist potency for a given receptor (i.e., GLP-2R). The EC₅₀ value refers to the concentration capable of producing 50% of the maximum effect. In the same assay for a specific receptor, a compound with a lower EC₅₀ value may be considered to have higher potency for the receptor.

The GLP-2R agonist activity of the GLP-2 derivative was assessed in the present disclosure. It was found that in the human GLP-2R reporter gene cell assay and the rat GLP-2R reporter gene cell assay, the activity of M2 and M3 was at least 3 times that of the control apraglutide.

Further, through rat pharmacokinetic studies and beagle dog pharmacokinetic studies, it was found that the M2 of the present disclosure had a significantly prolonged half-life and significantly improved plasma exposure level as compared with apraglutide. Specifically, in the rat pharmacokinetic studies, the half-lives of apraglutide and M2 were 7.3 h vs. 10.3 h, and the plasma exposure levels were 6620.0 h × nmol/L vs. 13265.1 h × nmol/L; in the beagle dog pharmacokinetic studies, the half-lives of apraglutide and M2 were 17.7 h vs. 54.8 h, and the plasma exposure levels were 4854.6 h × nmol/L vs. 25116.8 h × nmol/L.

Furthermore, the small intestine growth-promoting pharmacodynamic effect was evaluated in a rat small intestine growth model. The results show that M2 of the present disclosure can increase the weight and the coefficient of thickness of the small intestine in a dose-dependent manner, and exhibits significantly greater efficacy than apraglutide, where the efficacy of 10 nmol/kg M2 was between that of 50 nmol/kg and 100 nmol/kg apraglutide.

The GLP-2 derivative of the present disclosure was prepared into an oral tablet, and the oral tablet was orally administered to male beagle dogs at a dose of 1 tablet once daily for 5 consecutive days. It was found that 7 mg M2 achieved the maximum plasma exposure level of 62.4 nM on day 4, while the steady-state concentration of 7 mg semaglutide (Rybelsus), a glucagon-like peptide-1 (GLP-1) receptor agonist, in humans was about 7.5 nM. It can be seen that the bioavailability of oral M2 is substantially higher than that of Rybelsus.

### Pharmaceutical composition of GLP-2 derivative

The GLP-2 derivative of the present disclosure or the pharmaceutically acceptable salt thereof can be prepared into a pharmaceutical composition, wherein the GLP-2 derivative or the pharmaceutically acceptable salt thereof is present in a therapeutically effective amount.

The pharmaceutical composition described herein may be an oral delivery composition.

The pharmaceutical composition or the oral delivery composition described herein comprises a pharmaceutically acceptable excipient, in addition to the active ingredient GLP-2 derivative or the pharmaceutically acceptable salt thereof. Pharmaceutically acceptable excipients are well known to those skilled in the art, e.g., non-toxic fillers, stabilizers, diluents, carriers, solvents, and other formulation excipients, for example, diluents and excipients, such as microcrystalline cellulose, mannitol, etc.; fillers such as starch, sucrose, etc.; binders such as starch, cellulose derivatives, alginates, gelatin, and/or polyvinylpyrrolidone; disintegrants such as calcium carbonate and/or sodium bicarbonate; absorption enhancers such as quaternary ammonium compounds; surfactants such as cetyl alcohol; carriers and solvents, such as water, normal saline, kaolin, and bentonite; lubricants such as talc, calcium/magnesium stearate, and polyethylene glycol. In some embodiments, the absorption enhancer described in the present disclosure is an oral absorption enhancer or an oral delivery agent. The oral absorption enhancer or the oral delivery agent should be interpreted as any ingredient or a combination thereof well known to those skilled in the art that can enhance the oral absorption of polypeptides, e.g., an NAC salt, decanoate, Cu, Zn, or Fe ions, a reducing agent (e.g., ascorbic acid), tetrasodium ethylenediaminetetraacetate, sodium phosphate, tris(hydroxymethyl)aminomethane, lysine, or a combination thereof, or an oral delivery agent disclosed in US8753683B, CN104884078B, US7138546B, etc.; preferably, the oral delivery agent described in the present disclosure is an NAC salt (N-[8-(2-hydroxybenzoyl)amino]octanoate), such as a sodium or potassium salt thereof; further preferably, the NAC salt is a PNAC salt, i.e., potassium N-[8-(2-hydroxybenzoyl)amino]octanoate. The NAC salt or PNAC salt of the oral delivery agent described in the present application should be interpreted as any crystal form that can satisfy the oral delivery form of the composition of the present disclosure.

The NAC salt (N-[8-(2-hydroxybenzoyl)amino]octanoate) is disclosed in CN 116327890 B, the structural formula of which is represented by formula (II):

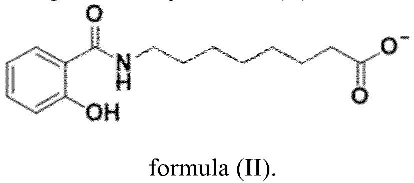

The NAC salt may be a sodium salt (SNAC) or a potassium salt (PNAC), preferably PNAC. The PNAC has a structural formula represented by formula (III):

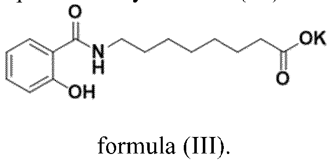

The preparation of NAC and PNAC has been disclosed in CN 116327890 B.

The oral delivery composition described in the present disclosure should be interpreted as any composition form that can be used for oral administration, e.g., the form of a solid, liquid, or semisolid. In some embodiments, the oral delivery composition described in the present application is in the form of a solid, such as a tablet, a capsule, a granule, or a pillet. Any solid form and proportion that can be used for the delivery of oral polypeptide compositions well known to those skilled in the art are within the scope of the present application.

In some embodiments, the GLP-2 derivative of the present disclosure is prepared into an oral tablet comprising a polypeptide derivative M2, a delivery agent (PNAC), and a lubricant (magnesium stearate), and the mass ratio of the three may be 7:(200-500):5-15.

The oral tablet described in the present disclosure may be prepared by a conventional method.

For example, the polypeptide derivative and PNAC are sieved, mixed well with other excipients, and directly tableted.

The derivative of the present disclosure or the pharmaceutically acceptable salt thereof may be used alone or in combination with any compound beneficial for promoting small intestine growth, preventing and/or treating obesity, and preventing and/or treating gastrointestinal disorders (e.g., a compound having an anti-inflammatory effect) in a pharmaceutical composition, which is expected to enhance beneficial therapeutic effects of the derivative of the present disclosure.

The pharmaceutical composition of the present disclosure may also be used in combination with one or more additional drugs. Such combined use is expected to achieve synergistic effects in the promotion of small intestine growth, prevention and/or treatment of obesity, and prevention and/or treatment of gastrointestinal disorders.

### Medical disorders

By administering an effective amount of the GLP-2 derivative or the salt thereof as described herein, the GLP-2 derivative of the present disclosure can be used as a pharmaceutical agent for preventing and/or treating obesity, and preventing or treating an individual with gastrointestinal disorders (including upper gastrointestinal disorders of the esophagus). The gastrointestinal disorders include: ulcer of any etiology (e.g., peptic ulcer, drug-induced ulcer, and ulcer associated with infection or other pathogens), digestive disorder, malnutrition (e.g., cachexia and anorexia), malabsorption syndrome, short bowel syndrome, blind loop syndrome, inflammatory bowel disease (e.g., Crohn's disease and ulcerative colitis), celiac sprue (e.g., caused by gluten-induced enteropathy or celiac disease), tropical sprue, hypogammaglobulinemia-type sprue, small intestine injury, chemotherapy-induced diarrhea/mucositis (CID), irritable bowel syndrome, and graft-versus-host disease.

Among these, short bowel syndrome (SBS), abbreviated as short gut, results from loss of intestinal absorption caused by surgical resection, congenital defects, or disease-associated conditions, in which patients are subsequently unable to maintain fluid, electrolyte, and nutrient balance on their regular diet. Although adaptation generally occurs within two years after resection, patients with SBS experience a decrease in dietary intake and fluid loss.

### Example 1: Preparation of Polypeptide Derivatives

The synthesis, cleavage, and purification of the polypeptide derivative were completed using a solid-phase organic synthesis method and an Fmoc-protected amino acid strategy to give a target product. Taking the polypeptide derivative M1 in Table 2 as an example, the preparation process was as follows:

### 1. Solid-phase synthesis:

Fmoc-Asp(OtBu)-Wang Resin (substitution = 0.35 mmol/g) was used with the Fmoc/tBu process. Based on the amino acid sequence of the polypeptide derivative described above, amino acids were sequentially coupled from the C-terminus to the N-terminus (from right to left) according to the method shown in Table 1 to ultimately form a linear polypeptide resin.

**Table 1. List of synthetic procedures**

| **No.** | **Solvent** | **Times** | **Time (min/times)** |
|---|---|---|---|
| 1 | 20% Pip/DMF | 1 | 10 |
| 2 | 20% Pip/DMF | 1 | 5 |
| 3 | DMF | 5 | 1 |
| 4 | Sampling and color development detection | | |
| 5 | AA/DIEA/TBTU | 1 | 60 |
| 6 | DMF | 4 | 1 |
| 7 | Sampling and color development detection | | |
| Procedure description | Repeat 1-7 until completion of the linear peptide synthesis | | |

The following amino acids were sequentially coupled:
A-01Fmoc-Thr(tBu)-OH, A-02Fmoc-Ile-OH, A-03Fmoc-Lys(Boc)-OH, A-04Fmoc-Thr(tBu)-OH, A-05Fmoc-Gln(Trt)-OH, A-06Fmoc-Ile-OH, A-07Fmoc-Leu-OH, A-08Fmoc-Trp(Boc)-OH, A-09Fmoc-Asn(Trt)-OH, A-10Fmoc-Ile-OH, A-11Fmoc-Phe-OH, A-12Fmoc-Asp(OtBu)-OH, A-13Fmoc-Arg(Pbf)-OH, A-14Fmoc-Ala-OH, A-15Fmoc-Ala-OH, A-16Fmoc-Leu-OH, A-17Fmoc-Leu-OH, A-18Fmoc-Asp(OtBu)-OH, A-19Fmoc-Leu-OH, A-20Fmoc-Ile-OH, A-21Fmoc-Thr(tBu)-OH,A-22Fmoc-Asn(Trt)-OH, A-23Fmoc-Met-OH, A-24Fmoc-Lys(Boc)-OH, A-25Fmoc-Asp(OtBu)-OH, A-26Fmoc-Ser(tBu)-OH, A-27Fmoc-Phe-OH, A-28Fmoc-Ser(tbu)-OH, A-29Fmoc-Gly-OH, A-30Fmoc-Asp(OtBu)-OH, A-31Fmoc-Gly-OH, A-32Boc-His(Trt)-OH

After the synthesis of the linear polypeptide resin was completed, Dde deprotection was performed using a hydrazine hydrate method, and the following building blocks were sequentially coupled:
B01Fmoc-AEEA-OH, B02Fmoc-AEEA-OH, B03Fmoc-Glu-OtBu, B04 C18 diacid-OtBu

The peptide resin was washed, transferred, and dried to constant weight for cleavage.

### 2. Cleavage of peptide resin:

Preparation of cleavage reagent: The volume of the cleavage reagent used was calculated using a ratio of 1 g of the peptide resin to 10 mL ± 2 mL of the cleavage reagent ( volume ratio of TFA:H₂O:EDT:TIS = 95:1:2:2). The required cleavage reagents H₂O, TFA, EDT, and TIS were sequentially placed into a cleavage reaction flask, and the temperature of the resulting cleavage reagent was controlled at 0-10 °C.

The cleavage reagent was added to the peptide resin under stirring. After the temperature of the system stabilized, the temperature was then controlled at 25-30 °C, and the mixture was stirred for 2.5 h. The cleavage solution was collected by filtration and subjected to precipitation using five-fold volumes of ice-cold diethyl ether relative to the volume of the filtrate. The precipitate was collected by filtration, washed 3 times using three-fold volumes of ice-cold diethyl ether relative to the volume of the filtrate, and dried at room temperature under reduced pressure to give a solid crude product.

### 3. Purification and lyophilization:

The crude product was finely ground, and purified water was prepared. The finely ground crude product was slowly added under stirring, while an aqueous acetonitrile solution was simultaneously added dropwise. After the crude product was added and dissolved completely, the mixture was filtered through a 0.45 µm microporous filter membrane. The crude product was purified using a C-18-packed preparative column (mobile phases, A: 0.1% TFA/H₂O; B: 0.1% TFA/ACN). Separation and purification were performed using an appropriate gradient at room temperature. The target product was collected, analyzed, detected, and classified. With an impurity purity requirement of ≥ 90%, the unqualified target substance was collected, and then separated and purified again using an appropriate gradient. The qualified main peak fractions were lyophilized under reduced pressure to give a refined polypeptide derivative in the form of a powder.

The polypeptide derivatives apraglutide, M2, M3, and M4 in Table 2 were synthesized according to similar methods, where apraglutide is a GLP-2 derivative in the clinical research stage developed by VectiveBio.

**Table 2. Polypeptide derivatives**

| Apraglutide (positive control) | HGDGS FSDE-Nle (D-phe)-TILD LLAAR DFINW LIQTK ITD-NH₂, SEQ ID NO: 1 |
|---|---|
| M1 | HGDGSFSDKMNTILDLLAARDFINWLIQTKITD, SEQ ID NO: 2 |
| | Lysine at position 9 was modified with (2-(2-(2-aminoethoxy)ethoxy)acetyl)₂-(γGlu)-CO-(CH₂)₁₆-CO₂H |
| M2 | HGDGSFSDEMNTILDKLAARDFINWLIQTKITD, SEQ ID NO: 3 |
| | Lysine at position 16 was modified with (2-(2-(2-aminoethoxy)ethoxy)acetyl)₂-(γGlu)-CO-(CH₂)₁₆-CO₂H |
| M3 | HGDGSFSDEMNTILDLLAARDFIKWLIQTKITD, SEQ ID NO: 4 |
| | Lysine at position 24 was modified with (2-(2-(2-aminoethoxy)ethoxy)acetyl)₂-(γGlu)-CO-(CH₂)₁₆-CO₂H |
| M4 | HGDGSFSDEMNTILDLLAARDFINWLIQTKITD, SEQ ID NO: 5 |
| | Lysine at position 30 was modified with (2-(2-(2-aminoethoxy)ethoxy)acetyl)₂-(γGlu)-CO-(CH₂)₁₆-CO₂H |

In Table 2, the ε amino group of lysine is linked to (2-(2-(2-aminoethoxy)ethoxy)acetyl)₂-(γGlu)-CO-(CH₂)₁₆-CO₂H via an amide bond; (2-(2-(2-Aminoethoxy)ethoxy)acetyl)₂-(γGlu)-CO-(CH₂)₁₆-CO₂H is 18-{[(23S)-23-carboxy-2,11,20-trioxo-10,19-diaza-4,7,13,16-tetraoxatricos-23-yl]amino}-18-oxooctadecanoic acid, the structural formula of which is represented by formula (I): 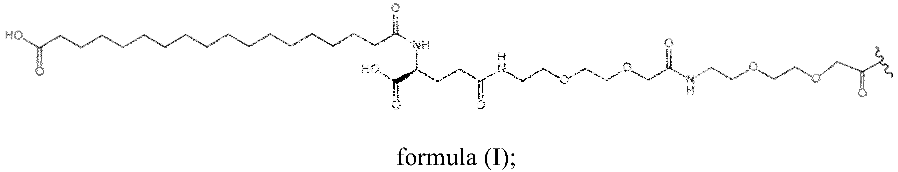 in formula (I), 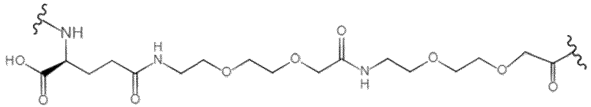 the moiety is a linker, 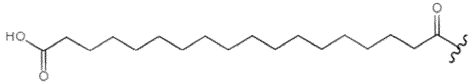 the moiety is a fatty acid, and the 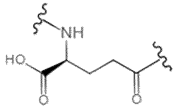 moiety in the linker is γGlu.

The abbreviations used in the preparation process described above have the following meanings:
AA: amino acid
AEEA: 2-(2-(2-aminoethoxy)ethoxy)acetic acid
Boc: t-butyloxycarbonyl
ACN: acetonitrile
DMF: N,N-dimethylformamide
DIEA: N,N-diisopropylethylamine
Dde: 2-(4,4-dimethyl-2,6-dioxocyclohexylidene)ethyl
EDT: 1,2-ethanedithiol
Fmoc: 9-fluorenylmethyloxycarbonyl
OtBu: tert-butyl ester group
Pbf: 2,2,4,6,7-pentamethyldihydrobenzofuran-5-sulfonyl chloride
Pip: piperidine
TBTU: O-(benzotriazol-1-yl)-N,N,N',N'-tetramethyluronium tetrafluoroborate
tBu: tertiary butyl
TFA: trifluoroacetic acid
TIS: triisopropylsilane
Trt: triphenylmethyl
yGlu: γ-glutamic acid

### Example 2: Cell Activity Assay of GLP-2 Derivatives

The activity of the GLP-2 derivatives in activating GLP-2R was investigated by human GLP-2R-CRE-Luciferase-HEK293 (human GLP-2R: NCBI reference sequence: NM_004246.3) or rat GLP-2R-CRE-Luciferase-HEK293 (rat GLP-2R: NCBI reference sequence: NM_021848.2) reporter gene assays.

Construction of human GLP-2R-CRE-Luciferase-HEK293: HEK293 cells (Cell Resource Center, Institute of Basic Medical Sciences, Peking Union Medical College, Chinese Academy of Medical Sciences, Catalog No. 1101HUM-PUMC000010) were transfected with plasmid pGL4.29 [luc2P/CRE/Hygro] vectors (Promega, Catalog No. E8471) containing multi-copy cAMP response element (CRE)-driven luciferase expression cassettes and pcDNA3.1(+) (Invitrogen, Catalog No. V79020) containing the human GLP-2R gene to obtain a transiently or stably transfected GLP-2R-CRE-Luciferase-HEK293 cell line containing luciferase expression plasmids.

### Human GLP-2R reporter gene cell assay:

In a 96-well plate, 100 µL of human GLP-2R-CRE-Luciferase-HEK293 cells (at a concentration of 3 × 10⁵ cells/mL) cultured in a DMEM + 10% FBS culture medium was added to each well. After 24 h of incubation in an incubator at 37 °C with 5% CO₂, the old culture medium was removed. 50 µL of fresh DMEM + 10% FBS culture medium was added, and 50 µL of apraglutide, M1, M2, M3, or M4 compound solution at different final concentrations was added to the corresponding wells. For the final concentration of each compound, ten 3-fold dilution gradients were set according to the descending concentration order of 2500 ng/mL, 833 ng/mL, and 278 ng/mL. After another 24 h of incubation in the incubator at 37 °C with 5% CO₂, 100 µL of a Bright-Glo reagent (Promega) was added, and the plate was gently tapped to facilitate solution mixing. After 3 minutes, chemiluminescence values were measured using a microplate reader. Four-parameter curve fitting was performed using GraphPad Prism 9, and EC₅₀ values were calculated.

### Rat GLP-2R reporter gene cell assay:

In a 96-well plate, 30,000 HEK293 cells cultured in a DMEM + 10% FBS culture medium were added to each well. The next day, 25 ng of a pcDNA3.1(+) plasmid containing the rat GLP-2R gene (Invitrogen, Catalog No. V79020) and 50 ng of a pGL4.29 [luc2P/CRE/Hygro] vector (Promega, Catalog No. E8471) were introduced into each well. After 24 h of incubation in an incubator at 37 °C with 5% CO₂, the old culture medium was removed. 50 µL of fresh DMEM + 10% FBS culture medium was added, and 50 µL of apraglutide, M2, or M3 compound solution at different final concentrations was added to the corresponding wells. For the final concentration of each compound, ten 3-fold dilution gradients were set according to the descending concentration order of 5000 ng/mL, 1667 ng/mL, and 556 ng/mL. After another 24 h of incubation in the incubator at 37 °C with 5% CO₂, 100 µL of a Bright-Glo reagent (Promega) was added, and the plate was gently tapped to facilitate solution mixing. After 3 minutes, chemiluminescence values were measured using a microplate reader. Four-parameter curve fitting was performed using GraphPad Prism 9, and EC₅₀ values were calculated.

The results are shown in Table 3.

**Table 3. Cell viability assay**

| Sample | Human GLP-2R reporter gene cell assay, EC₅₀ (nM) | Rat GLP-2R reporter gene cell assay, EC₅₀ (nM) |
|---|---|---|
| Apraglutide | 2.11 | 1.07 |
| M1 | 4.32 | ND |
| M2 | 0.68 | 0.17 |
| M3 | 0.79 | 0.09 |
| M4 | 210.85 | ND |
| In Table 3, ND: not detected. | | |

The results showed that for the activity in activating GLP-2R:
In the human GLP-2R cell assay, the activity of M2 and M3 was about three times that of the control molecule apraglutide, while the activity of M1 was about half that of apraglutide, and the activity of M4 was nearly 100-fold less than that of apraglutide. The results indicate that the fatty acid modification at both positions 16 and 24 contributes to maintaining high molecular activity, while the fatty acid modification at both positions 9 and 30 reduces molecular activity to different degrees.

In the rat GLP-2R cell assay, both M2 and M3 exhibited stronger activity than the control molecule apraglutide.

### Example 3: Rat Pharmacokinetic Study

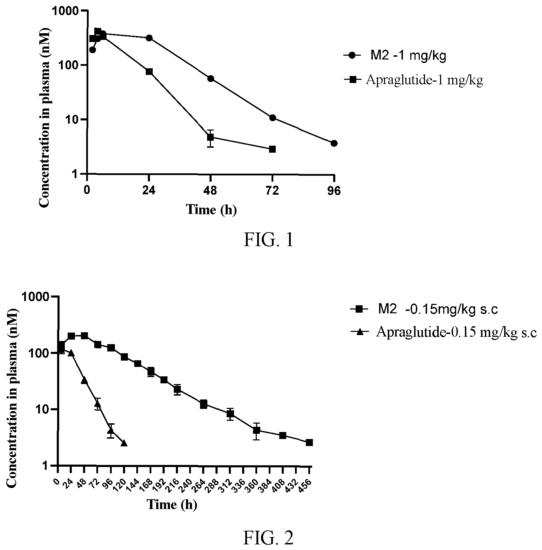

Male SD rats aged about 10 weeks were used in this study. The housing conditions for the experimental animals were a room temperature at 20 °C-23 °C and a relative humidity of 40%-50%. During the animal quarantine period and throughout the experimental process, the feed used was the feed 1035 for the growth and reproduction of rats for the Co60 irradiation experiment, the drinking water was purified water in drinking bottles, and the animals had free access to water. A drug molecule (i.e., apraglutide or M2) (n = 3) was administered via a single subcutaneous injection at a dose of 1 mg/kg (the solvent was PBS, pH 7.4; the drug concentration was 0.2 mg/mL; and the administration volume was 5 mL/kg). Whole blood samples were collected from the animals before administration (-5 min) and 2 h, 4 h, 6 h, 24 h, 48 h, 72 h, and 96 h after administration, and plasma samples were prepared (EDTA was used as the anticoagulant). The plasma samples were stored at -80 °C and used for subsequent analysis. The drug content in the plasma samples was analyzed by LC-MS/MS (Waters ACQUITY I Class Premier UPLC tandem with Sciex 6500 + QQQ). The ion transition charge-to-mass ratio selected for apraglutide was 942.2/1178.2, and the ion transition charge-to-mass ratio selected for M2 was 1121.3/1088.3. The experimental data were plotted using GraphPad Prism 10. The rat pharmacokinetic profile is shown in FIG. 1.

Pharmacokinetic parameters (Cₘₐₓ, Tₘₐₓ, T_{1/2}, AUC, and MRT) of the drug were calculated using a non-compartmental model. The results are shown in Table 4.

**Table 4. Pharmacokinetic parameters**

| Parameter | Apraglutide | M2 |
|---|---|---|
| Cₘₐₓ (nmol/L) | 425.2 | 382.7 |
| Tₘₐₓ (h) | 4 | 6 |
| T_{1/2} (h) | 7.3 | 10.3 |
| AUC_{0-inf obs} (h × nmol/L) | 6620.0 | 13265.1 |
| MRT_{0-inf obs} (h) | 10.8 | 20.9 |

The results showed that in SD rats, compared with apraglutide, M2 exhibited a longer half-life and higher plasma exposure level, further confirming that the introduction of a fatty acid side chain into the molecule could significantly improve the pharmacokinetic properties of the molecule.

### Example 4: Beagle Dog Pharmacokinetic Study

In this study, male beagle dogs weighing 8-12 kg were used, and a drug molecule (i.e., apraglutide or M2) (n = 2) was administered via a single subcutaneous injection at a dose of 0.15 mg/kg (the solvent was PBS, pH 7.4; the drug concentration was 0.3 mg/mL; and the single injection volume did not exceed 2 mL). Whole blood samples were collected from the animals before administration (-10 min) and 6 h, 24 h, 48 h, 72 h, 96 h, 120 h, 144 h, 168 h, 192 h, 216 h, 264 h, 312 h, 360 h, 408 h, and 456 h after administration, and plasma samples were prepared (heparin sodium was used as the anticoagulant). The plasma samples were stored at -80 °C and used for subsequent analysis. The drug content in the plasma samples was analyzed by LC-MS/MS (Waters ACQUITYI Class Premier UPLC tandem with Sciex 6500 + QQQ). The ion transition charge-to-mass ratio selected for apraglutide was 942.2/1178.2, and the ion transition charge-to-mass ratio selected for M2 was 1121.3/1088.3. The experimental data were plotted using GraphPad Prism 10. The beagle dog pharmacokinetic profile is shown in FIG. 2.

Pharmacokinetic parameters (Cₘₐₓ, Tₘₐₓ, T_{1/2}, AUC, and MRT) of the drug were calculated using a non-compartmental model. The results are shown in Table 5.

**Table 5. Pharmacokinetic parameters**

| Parameter | Apraglutide | M2 |
|---|---|---|
| Cₘₐₓ (nmol/L) | 117.0 | 203.8 |
| Tₘₐₓ (h) | 6 | 48 |
| T_{1/2} (h) | 17.7 | 54.8 |
| AUC_{0-inf obs} (h × nmol/L) | 4854.6 | 25116.8 |
| MRT_{0-inf obs} (h) | 29.6 | 93.8 |

The results showed that in beagle dogs, compared with apraglutide, M2 exhibited a longer half-life and higher plasma exposure level, further confirming that the introduction of a fatty acid side chain into the molecule can significantly improve the pharmacokinetic properties of the molecule.

### Example 5: Evaluation of GLP-2 Derivative in Rat Small Intestine Growth Model

SD rats (aged 6-8 weeks, male, weighing about 200 g, SPF (Beijing) Biotechnology Co., Ltd.) were used as experimental subjects in this study. The animals were randomly and evenly grouped based on body weight, with 5 animals in each group. The experimental animals were subcutaneously administered with vehicle (vehicle control, PBS, pH 7.4), apraglutide (10/50/250 nmol/kg), and M2 (10/50/250 nmol/kg) according to a standard of 5 mL/kg once daily for five consecutive days. After the final administration, all experimental animals were fasted and weighed after 24 h. Subsequently, the experimental animals were anesthetized and sacrificed, and the small intestine (from the pylorus to the anterior end of the cecum) was collected, rinsed with normal saline, and blotted dry with gauze. Then, the length of the small intestine was measured, and the small intestine was weighed. Thus, the growth-promoting effects of apraglutide and M2 on the small intestine of SD rats were evaluated. The coefficient of thickness was the ratio of the weight of the small intestine (g) to the length of the small intestine (cm). Plotting was performed using GraphPad Prism 10. The mean ± standard error (SEM) was used to show the weight or coefficient of thickness of the small intestine of each group, and the differences between the groups were compared. Statistical differences were analyzed by One-way ANOVA. Compared with the vehicle control group, ** indicated p < 0.01, **** indicated p < 0.0001, and ns indicated no statistical difference.

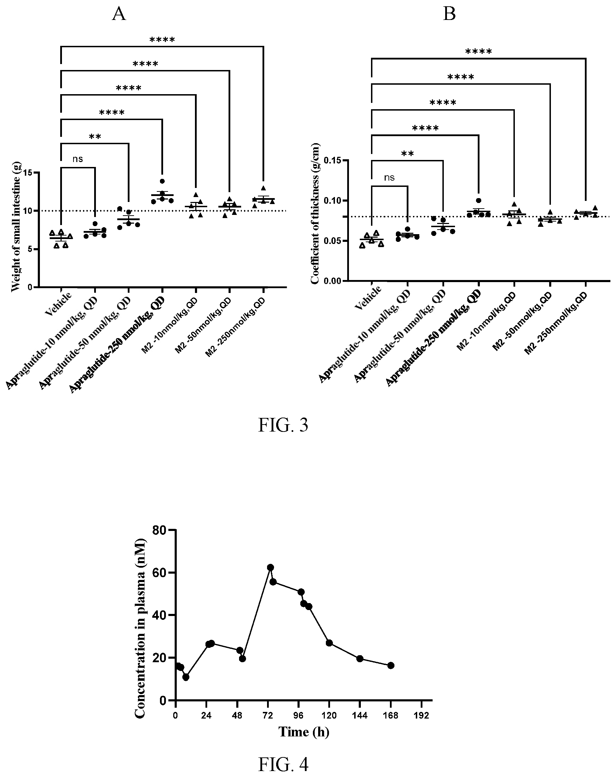

The small intestine growth-promoting pharmacodynamic study results are shown in FIG. 3.

The results showed that compared with the vehicle control, both apraglutide and M2 could increase the weight and the coefficient of thickness of the small intestine in a dose-dependent manner. The efficacy of 10 nmol/kg M2 was between that of 50 nmol/kg and 250 nmol/kg apraglutide, which was attributed to the higher GLP-2R activation activity and longer half-life of M2.

### Example 6: Pharmacokinetic Study of Oral GLP-2 Derivative in Beagle Dogs

### Preparation of oral tablets:

The content of certain main ingredients in polypeptide derivative tablets containing fatty acid side chains is shown in Table 6:

**Table 6. Polypeptide derivative tablets**

| Formulation composition | Polypeptide derivative M2 (mg/tablet) | Delivery agent (PNAC) (mg/tablet) | Magnesium stearate (mg/tablet) |
|---|---|---|---|
| 1 tablet | 7 | 300 | 8 |

The preparation method for the polypeptide derivative tablets containing fatty acid side chains was as follows: The polypeptide derivative and PNAC (potassium N-[8-(2-hydroxybenzoyl)amino]octanoate) were sieved, mixed well with excipients, and directly tableted.

### Animal experimental scheme:

Male beagle dogs (9-12 kg) aged 10-15 months were orally administered with 1 drug tablet (M2) (n = 5) once daily for 5 consecutive days. The first oral administration was recorded as Day 1, and the final oral administration was recorded as Day 5. On Day 1 of administration, whole blood samples were collected from the animals before administration (-10 min) and 2 h, 4 h, and 8 h after administration, and plasma samples were prepared (sodium heparin was used as the anticoagulant). On Day 2 to Day 4 of administration, whole blood samples were collected from the animals 2 h and 4 h after administration, and plasma samples were prepared (sodium heparin was used as the anticoagulant). On Day 5 of administration, whole blood samples were collected from the animals 2 h, 4 h, 8 h, 24 h, 48 h, and 72 h after administration, and plasma samples were prepared (sodium heparin was used as the anticoagulant). The plasma samples were stored at - 80 °C and used for subsequent analysis. The drug content in the plasma samples was analyzed by LC-MS/MS (Waters ACQUITYI Class Premier UPLC tandem with Sciex 6500 + QQQ). The ion transition charge-to-mass ratio selected for M2 was 1121.3/1088.3. The experimental data were plotted using GraphPad Prism 10. The beagle dog pharmacokinetic profile after oral administration is shown in FIG. 4.

The results showed that in the beagle dog oral administration study, after 5 consecutive days of administration, 7 mg M2 reached the maximum plasma exposure level of 62.4 nM on Day 4, while the steady-state concentration of 7 mg semaglutide (Rybelsus) in humans was about 7.5 nM (Assessment Report EMA/95374/2020). It can be seen that the bioavailability of oral M2 is substantially higher than that of Rybelsus, indicating the feasibility of oral administration of M2.

## Claims

1. A glucagon-like peptide-2 derivative or a pharmaceutically acceptable salt thereof, wherein the amino acid sequence of the glucagon-like peptide-2 derivative is:
HGDGSFSDEMNTILDX₁₆LAARDFIX₂₄WLIQTKITD,
wherein X₁₆ is selected from K and L, X₂₄ is selected from N and K, and at least one of X₁₆ and X₂₄ is K;
a fatty acid side chain is linked to the amino acid K residue at position 16 and/or 24 of the derivative.

2. The glucagon-like peptide-2 derivative or the pharmaceutically acceptable salt thereof according to claim 1, wherein X₁₆ is K, and X₂₄ is N.

3. The glucagon-like peptide-2 derivative or the pharmaceutically acceptable salt thereof according to claim 1, wherein X₁₆ is L, and X₂₄ is K.

4. The glucagon-like peptide-2 derivative or the pharmaceutically acceptable salt thereof according to any one of claims 1-3, wherein the derivative is linked to the fatty acid side chain via the ε amino group of the amino acid K residue at position 16 and/or 24.

5. The glucagon-like peptide-2 derivative or the pharmaceutically acceptable salt thereof according to any one of claims 1-4, wherein the fatty acid side chain is selected from one or more of 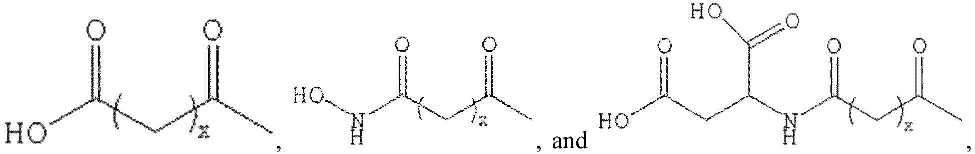
wherein x is any integer from 4 to 38;
preferably, the fatty acid side chain is selected from one or more of:
HOOC(CH₂)₁₄CO-, HOOC(CH₂)₁₅CO-, HOOC(CH₂)₁₆CO-, HOOC(CH₂)₁₇CO-, HOOC(CH₂)₁₈CO-, HOOC(CH₂)₁₉CO-, HOOC(CH₂)₂₀CO-, HOOC(CH₂)₂₁CO-, and HOOC(CH₂)₂₂CO-.

6. The glucagon-like peptide-2 derivative or the pharmaceutically acceptable salt thereof according to any one of claims 1-5, wherein the fatty acid side chain is linked to the amino acid K residue at position 16 and/or 24 of the derivative via a linker.

7. The glucagon-like peptide-2 derivative or the pharmaceutically acceptable salt thereof according to claim 6, wherein the linker is selected from one or more of 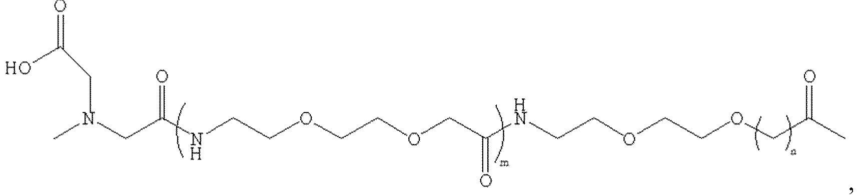 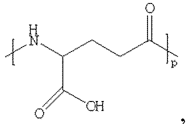 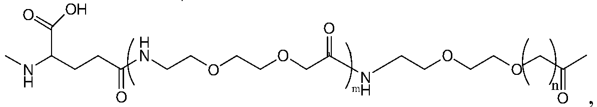 and 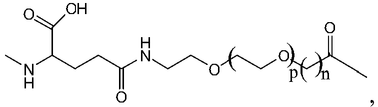 wherein m is 0, 1, 2, or 3; n is 1 or 2; p is any integer from 1 to 5;
preferably, the linker is: 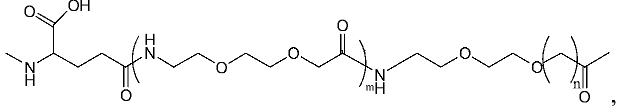 wherein m is 0, 1, 2, or 3, and n is 1; more preferably, m is 1, and n is 1.

8. A method for preparing the glucagon-like peptide-2 derivative or the pharmaceutically acceptable salt thereof according to any one of claims 1-7;
preferably, the preparation method comprises a step of preparing the glucagon-like peptide-2 derivative using a chemical method and/or a biological method;
preferably, the chemical method comprises liquid-phase or solid-phase polypeptide synthesis.

9. A pharmaceutical composition, comprising the glucagon-like peptide-2 derivative or the pharmaceutically acceptable salt thereof according to any one of claims 1-7, and a pharmaceutically acceptable excipient;
preferably, the pharmaceutical composition is in the form of a solid, liquid, or semisolid;
preferably, the pharmaceutical composition is an oral delivery composition; preferably, the oral delivery composition is in the form of a solid, liquid, or semisolid, more preferably a solid, and further preferably an oral tablet;
preferably, the oral delivery composition further comprises an oral absorption enhancer;
preferably, the oral absorption enhancer is selected from one or more of the following: an NAC salt, decanoate, Cu, Zn, or Fe ions, a reducing agent, tetrasodium ethylenediaminetetraacetate, sodium phosphate, tris(hydroxymethyl)aminomethane, and lysine; the reducing agent is preferably ascorbic acid; preferably, the oral absorption enhancer is an NAC salt (N-[8-(2-hydroxybenzoyl)amino]octanoate), and the NAC salt is preferably one or more of a sodium salt (SNAC) and a potassium salt (PNAC).

10. Use of the glucagon-like peptide-2 derivative or the pharmaceutically acceptable salt thereof according to any one of claims 1-7, or the pharmaceutical composition according to claim 9 in the manufacture of a medicament for promoting small intestine growth, preventing and/or treating obesity, and preventing and/or treating a gastrointestinal disorder;
preferably, the gastrointestinal disorder is ulcer, digestive disorder, malnutrition, malabsorption syndrome, short bowel syndrome, blind loop syndrome, inflammatory bowel disease, celiac sprue, tropical sprue, hypogammaglobulinemia-type sprue, small intestine injury, chemotherapy-induced diarrhea/mucositis, irritable bowel syndrome, or graft-versus-host disease; preferably, the inflammatory bowel disease is Crohn's disease or ulcerative colitis;
preferably, the medicament is an oral drug;
preferably, the oral drug is in the form of a solid, liquid, or semisolid, more preferably a solid, and further preferably an oral tablet.

11. The glucagon-like peptide-2 derivative or the pharmaceutically acceptable salt thereof according to any one of claims 1-7, or the pharmaceutical composition according to claim 9 for use in therapy.

12. A method for promoting small intestine growth, preventing and/or treating obesity, and preventing and/or treating a gastrointestinal disorder, comprising a step of administering to a patient in need thereof a therapeutically effective amount of the glucagon-like peptide-2 derivative or the pharmaceutically acceptable salt thereof according to any one of claims 1-7 or the pharmaceutical composition according to claim 9;
preferably, the gastrointestinal disorder is ulcer, digestive disorder, malnutrition, malabsorption syndrome, short bowel syndrome, blind loop syndrome, inflammatory bowel disease, celiac sprue, tropical sprue, hypogammaglobulinemia-type sprue, small intestine injury, chemotherapy-induced diarrhea/mucositis, irritable bowel syndrome, or graft-versus-host disease; preferably, the inflammatory bowel disease is Crohn's disease or ulcerative colitis.
